(19) Europäisches Patentamt
European Patent Office
Office européen des brevets

(11) Veröffentlichungsnummer: **0 314 984 B1**

# (12) EUROPÄISCHE PATENTSCHRIFT

(45) Veröffentlichungstag der Patentschrift: **02.12.92**

(51) Int. Cl.[5]: **A61K 31/505**

(21) Anmeldenummer: **88117454.4**

(22) Anmeldetag: **20.10.88**

## (54) Verwendung von 2-Pyrimidinyl-1-piperazin-Derivaten.

(30) Priorität: **31.10.87 DE 3736974**

(43) Veröffentlichungstag der Anmeldung:
**10.05.89 Patentblatt 89/19**

(45) Bekanntmachung des Hinweises auf die Patenterteilung:
**02.12.92 Patentblatt 92/49**

(84) Benannte Vertragsstaaten:
**AT BE CH DE ES FR GB IT LI LU NL SE**

(56) Entgegenhaltungen:
**EP-A- 129 128**
**EP-A- 0 285 008**

**DRUG AND ALCOHOL DEPENDENCE, Band 21, Mai 1988, Elsevier Scientific Publishers Ireland, Ltd. (IR); K.OPTIZ et al., Seiten 99-104***

**ARCH. GEN. PSYCHIATRY, Band 46, März 1989; GAWIN et al., Seiten 288-289***

**CLIN. PHARMACY, Band 8, Oktober 1989; C.L.NUNN-THOMPSON et al., Seiten 710-720***

(73) Patentinhaber: **Troponwerke GmbH & Co. KG**
**Berliner Strasse 156**
**W-5000 Köln 80(DE)**

(72) Erfinder: **Opitz, Klaus, Prof. Dr.**
**Goerlitzer Strasse 102**
**W-4000 Muenster(DE)**
Erfinder: **Weischer, Maria-Luise, Dr.**
**Kanalstrasse 407**
**W-4000 Muenster(DE)**
Erfinder: **Traber, Jörg, Dr.**
**Loewenburgstrasse 12**
**W-5204 Lohmar 21(DE)**

(74) Vertreter: **Dänner, Klaus, Dr. et al**
**c/o Bayer AG Konzernverwaltung RP Patentabteilung**
**W-5090 Leverkusen 1 Bayerwerk(DE)**



Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

## Beschreibung

Die Erfindung betrifft die Verwendung von 2-Pyrimidinyl-1-piperazin-Derivatenzur Herstellung von Arzneimitteln für die Behandlung der Abhängigkeit von Nikotin.

Aus der EP-A-0 129 128 sind 2-Pyrimidinyl-1-piperazin-Derivate und ihre im wesentlichen anxiolytische Wirkung bekannt. Bekannte Wirkstoffe aus dieser Substanzklasse sind 8[4-N[4-(2-Pyrimidinyl)-1-piperazinyl]butyl]-8-azaspiro[4.5]-decan-7,9-dion-hydrochlorid (nach INN: Buspiron, Pharmacol. Biochem. Behav. 23, 687 bis 694 (1985)), 4,4-Dimethyl-1-[4-[4-(2-pyrimidinyl)-1-piperazinyl]butyl]-2,6-piperidindion-hydrochlorid (nach INN: Gepiron, Naunyn-Schmiedeberg's Arch. Pharmacol. 335, 454 bis 464 (1987)) und 2-(4-(4-(2-Pyrimidinyl)-1-piperazinyl)-butyl)-1,2-benzoisothiazol-3(2H)on-1,1-dioxidhydrochlorid (nach INN: Ipsapiron, Naunyn-Schmiedeberg's Arch. Pharmacol. 328, 467 bis 470 (1985)).

Die Nikotinabhängigkeit äußert sich in dem starken Rauchverlangen, dem Auftreten von Entzugserscheinungen und dem Unvermögen der meisten Raucher, die lebensbedrohende "Gewohnheit" aufzugeben.

Versuche, das zwanghafte Rauchverlangen (Craving) der abhängigen Raucher in der Abstinenz durch Haferextrakte, Lobelin oder Cytisin zu stillen, war wenig erfolgreich (Dtsch. med. Wschr. 112, 559-564 (1987)). Der Einsatz von Nikotin selbst, z.B. in Form von Kaugummi, führt zu einer mäßigen Verbesserung der Erfolgsquote von Raucherentwöhnungen (Lancet 2, 27-30 (1987)). Diese Methode der Raucherentwöhnung ist jedoch problematisch, da Nikotin ein starkes Gift ist und abhängig macht (J. Amer. med. Assoc. 255, 3277-3279 (1986)).

Es wurde die Verwendung von 2-Pyrimidinyl-1-piperazin-Derivaten der Formel

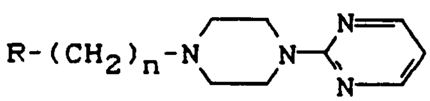

(I)

in der

| | |
|---|---|
| n | für eine der Zahlen 2, 3, 4, 5 oder 6 steht und |
| R | für einen der Reste |

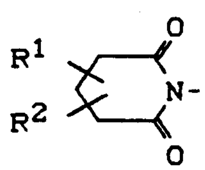

oder

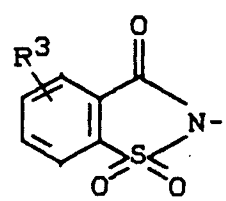

oder

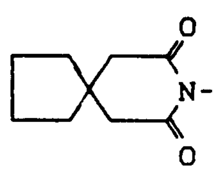

worin

| | |
|---|---|
| $R^1$, $R^2$ und $R^3$ | gleich oder verschieden sind und Wasserstoff oder einen geradkettigen oder verzweigten Kohlenwasserstoffrest mit 1 bis 6 Kohlenstoffatomen bedeuten |

steht,

und/oder deren Salze

zur Herstellung von Arzneimitteln zur Behandlung der Abhängigkeit von Nikotin gefunden.

Im Rahmen der Formel (I) bedeutet ein geradkettiger oder verzweigter Kohlenwasserstoffrest mit 1 bis 6 Kohlenstoffatomen beispielsweise Methyl, Ethyl, Propyl, Isopropyl, Butyl, Isobutyl, Pentyl, Isopentyl, Hexyl oder Isohexyl.

Bevorzugt werden Methyl und Ethyl.

Bevorzugt werden 2-Pyrimidinyl-1-piperazin-Derivate der Formel (I), wobei

| | |
|---|---|
| n | für eine der Zahlen 3 oder 4 steht und |
| $R^1$, $R^2$ und $R^3$ | Wasserstoff oder Methyl bedeuten. |

Als Salze seien pharmakologisch unbedenkliche Salze, wie die Hydrochloride, genannt.

Insbesondere bevorzugt werden Ipsapiron, Gepiron und Buspiron.

Die Herstellung der 2-Pyrimidinyl-1-piperazin-Derivate ist an sich bekannt (DE-A-33 21 969) und kann beispielsweise durch Umsetzung von entsprechenden Benzisothiazolen mit (Piperazinyl)-pyrimidinen erfolgen.

Die erfindungsgemäßen Arzneimittel enthalten im allgemeinen 1 bis 15 Gew.-%, bevorzugt 5 bis 10 Gew.-% an 2-Pyrimidinyl-1-piperazin-Derivaten.

Es ist selbstverständlich möglich, daß die erfindungsgemäßen Arzneimittel weitere an sich bekannte Wirkstoffe enthalten.

Die erfindungsgemäßen Arzneimittel können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht-toxischer, pharmazeutisch geeigneter Trägerstoffe oder Lösungsmittel.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Falle der Benutzung von Wasser als Verdünnungsmittel gegebenenfalls organische Lösungsmittel als Hilfslösungsmittel verwendet werden können.

Als Hilfsstoffe seien beispielsweise aufgeführt: Wasser, nicht-toxische organische Lösungsmittel, wie Paraffine (z.B. Erdölfraktionen), pflanzliche Öle (z.B. Erdnuß/Sesamöl), Alkohole (z.B. Ethylalkohol, Glycerin), Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Rohr-, Milch- und Traubenzucker), Emulgiermittel (z.B. Polyoxyethylen-Fettsäure-Ester, Polyoxyethylen-Fettalkohol-Ether, Alkylsulfonate und Arylsulfonate), Dispergiermittel (z.B. Lignin, Sulfitablaugen, Methylzellulose, Stärke und Polyvinylpyrrolidon) und Gleitmittel (z.B. Magnesiumstearat, Talkum, Stearinsäure und Natriumlaurylsulfat).

Die Applikation erfolgt in üblicher Weise, vorzugsweise oral, parenteral, perlingual oder intravenös. Im Falle der oralen Anwendung können Tabletten selbstverständlich außer den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen können die Wirkstoffe außer den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden. Im allgemeinen hat es sich als vorteilhaft erwiesen, bei intravenöser Applikation Mengen von 0,001 bis 1 mg/kg, vorzugsweise 0,01 bis 0,5 mg/kg Körpergewicht zur Erzielung wirksamer Ergebnisse zu verabreichen, und bei oraler Applikation beträgt die Dosierung 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchen die Verabreichung erfolgt. So kann es in einigen Fällen ausreichen sein, mit weniger als der vorgenannten Mindestmenge auszukommen, wenn in anderen Fällen die genannte obere Grenze überschritten werden muß. Im Falle der Applikation größerer Mengen kann es empfehlenswert sein, diese in mehreren Einzelgaben über den Tag zu verteilen.

Die erfindungsgemäßen 2-Pyrimidinyl-1-piperazin-Derivate beseitigen das zwanghafte Rauchverlangen und erleichtern somit den Nikotinentzug.

Beispiel 1 (Herstellung)

Darstellung von 2-(4-(4-(2-Pyrimidinyl)-1-piperazinyl)-butyl)-1,2-benzoisothiazol-3(2H)on-1,1-dioxid

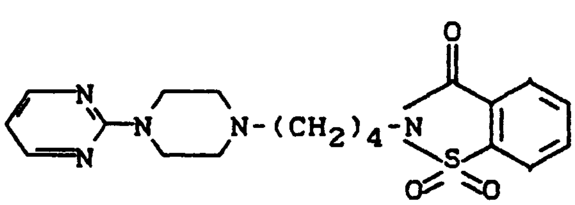

0,02 Mol 2-(4-Brombutyl)-1,2-benzoisothiazol-3-(2H)on-1,1-dioxid und 0,02 Mol 1-(2-Pyrimidyl)-piperazin werden mit 0,02 Mol $K_2CO_3$ in 150 ml absolutem Dimethylformamid (DMF) 1 Stunde bei 100 °C gerührt. Danach engt man ein. Mit Wasser versetzt, wird die organische Substanz in Methylenchlorid ($CH_2Cl_2$) aufgenommen. Die getrocknete $CH_2Cl_2$-Phase wird auf eine Kieselgelsäule gegeben und mit $CH_2Cl_2/CH_3OH$ (95:5) eluiert.

Ausbeute: 34 % der Theorie; Fp.: 138-139 °C.

Beispiel 2 (Anwendung)

Die untersuchten Tupaias (Tupaia belangeri) bevorzugen bei freier Wahl eine wäßrige Nikotin-Hydrogentartrat-Lösung (10 mg/l) gegenüber Wasser.

In der Untersuchung haben 10 Tupaias die freie Wahl der Nikotin-Aufnahme. Die Nikotin-Lösung wird einmal täglich an 5 aufeinanderfolgenden Tagen in der angegebenen Konzentration den Tieren angeboten, wobei sie immer auch die Wahl haben, nikotinfreies Wasser zu trinken. Die orale Verabreichung des Ipsapirons erfolgte jeweils 15 bis 30 Minuten vor Beginn der an jedem der 5 Tage stattfindenden Nikotin-Exposition.

Tabelle 1 zeigt den Einfluß von Ipsapiron auf die freiwillige Nikotin-Aufnahme.

Tabelle 1

| Wirkstoff | Wirkstoff-Dosis (mg/kg p.o.) | N % | Signifikanz |
|---|---|---|---|
| Ipsapiron | 15<br>30 | -32,3<br>-25,9 | p < 0,0003<br>p < 0,0002 |
| N bedeutet die abnahame der Nikotin-Ingestion in Prozent nach Behandlung gegenüber dem Konsum der unbehandelten Tiere in der vorangegangenen Woche (100 %) | | | |

Die Signifikanzen wurden mit Hilfe von Student's t-Test für gepaarte Werte errechnet.

Die Untersuchung zeigt, daß der freiwillige Nikotinkonsum nach Behandlung mit dem Wirkstoff erheblich abnimmt.

**Patentansprüche**

**1.** Verwendung von 2-Pyrimidinyl-1-piperazin-Derivaten der Formel

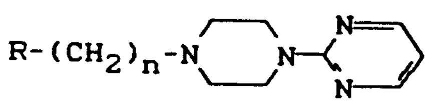

(I)

in der

n für eine der Zahlen 2, 3, 4, 5 oder 6 steht und

R für einen der Reste

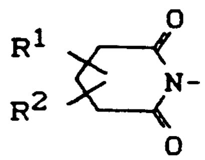 oder 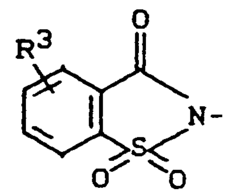 oder

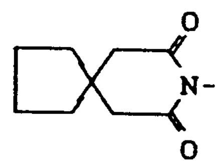 steht,

worin

$R^1$, $R^2$ und $R^3$ gleich oder verschieden sind und Wasserstoff oder einen geradkettigen oder verzweigten Kohlenwasserstoffrest Mit 1 bis 6 Kohlenstoffatomen bedeuten

und/oder deren Salze

zur Herstellung von Arzneimitteln zur Behandlung der Abhängigkeit von Nikotin.

**2.** Verwendung nach Anspruch 1, dadurch gekennzeichnet, daß als 2-Pyrimidinyl-1-piperazin-Derivat Ipsapiron eingesetzt wird.

**3.** Verwendung nach Anspruch 1, dadurch gekennzeichnet, daß als 2-Pyrimidinyl-1-piperazin-Derivat Gepiron eingesetzt wird.

**4.** Verwendung nach Anspruch 1, dadurch gekennzeichnet, daß als 2-Pyrimidinyl-1-piperazin-Derivat Buspiron eingesetzt wird.

**5.** Verwendung nach den Ansprüchen 1 bis 4, dadurch gekennzeichnet, daß das Arzneimittel 1 bis 15 Gew.-% an 2-Pyrimidinyl-1-piperazin-Derivaten enthält.

**Claims**

**1.** Use of 2-pyrimidinyl-1-piperazine derivatives of the formula

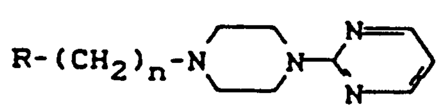 (I)

in which

n represents one of the numbers 2, 3, 4, 5 or 6 and

r represents one of the radicals

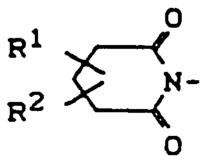 or 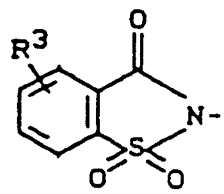 or

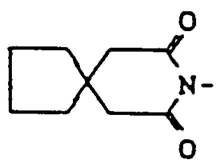

in which
$R_1$, $R_2$ amd $R_3$ are identical or different and denote hydrogen or a straight-chain or branched hydrocarbon radical having one to 6 carbon atoms
and/or their salts
for the production of medicaments for the treatment of nicotine dependence.

**2.** Use according to Claim 1, characterised in that the 2-pyrimidinyl-1-piperazine derivative employed is ipsapirone.

**3.** Use according to Claim 1, characterised in that the 2-pyrimidinyl-1-piperazine derivative employed is gepirone.

**4.** Use according to Claim 1, characterised in that the 2-pyrimidinyl-1-piperazine derivative employed is buspirone.

**5.** Use according to Claims 1 to 4, characterised in that the medicament contains 1 to 15 % by weight of 2-pyrimidinyl-1-piperazine derivatives.

## Revendications

**1.** Utilisation de dérivés de 2-pyrimidinyl-1-pipérazine de formule

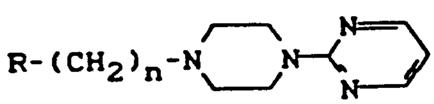 (I)

dans laquelle

n représente l'un des nombres 2, 3, 4, 5 et 6 et

R représente l'un des restes

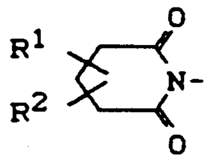 ou 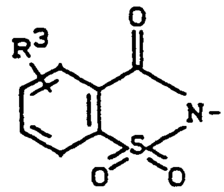 ou

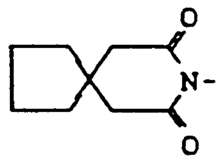

où

$R^1$, $R^2$ et $R^3$ sont identiques ou différents et représentent l'hydrogène ou un reste d'hydrocarbure à chaîne droite ou ramifiée ayant 1 à 6 atomes de carbone

et/ou de leurs sels

pour la préparation de médicaments destinés à traiter la dépendance à l'égard de la nicotine.

**2.** Utilisation suivant la revendication 1, caractérisée en ce qu'on utilise l'ipsapirone comme dérivé de 2-pyrimidinyl-1-pipérazine.

**3.** Utilisation suivant la revendication 1, caractérisée en ce qu'on utilise la gépirone comme dérivé de 2-pyrimidinyl-1-pipérazine.

**4.** Utilisation suivant la revendication 1, caractérisée en ce qu'on utilise la buspirone comme dérivé de 2-pyrimidinyl-1-pipérazine.

**5.** Utilisation suivant les revendications 1 à 4, caractérisée en ce que le médicament contient 1 à 15 % en poids de dérivés de 2-pyrimidinyl-1-pipérazine.